# EUROPEAN PATENT APPLICATION

(11) **EP 3 742 157 A1**
(43) Date of publication of application: **25.11.2020**
(21) Application number: 18901115.8
(22) Date of filing: 20.12.2018
(51) Int. Cl.: G01N 27/02, C12M 1/34, C12Q 1/06, G01N 15/00

(54) **CELL TESTING DEVICE, CELL TESTING METHOD, PROGRAM, AND RECORDING MEDIUM**

(30) Priority: 17.01.2018 JP 2018005648
(71) Applicant: Yokogawa Electric Corporation, Tokyo 180-8750 (JP)
(72) Inventor: HIRANO, Akinari, Musashino-shi, Tokyo 180-8750 (JP)
(74) Representative: Winter, Brandl, Fürniss, Hübner, Röss, Kaiser, Polte - Partnerschaft mbB
(86) International application number: PCT/JP2018/047018
(87) International publication number: WO 2019/142590

(57) **Abstract**

A cell testing device according to one aspect of the present invention includes: an impedance sensor configured to measure impedance of a culture solution; a storage unit configured to store a coefficient for estimating the number of viable cells present in the culture solution during a prescribed period using the impedance for each of a plurality of periods into which the prescribed period within a culture period from the start of cell culture to death is divided; and a viable cell count estimation unit configured to acquire the impedance and estimate the number of viable cells using at least one of the acquired impedance and a coefficient for each period stored in the storage unit.

## Description

### [Technical Field]

The present invention relates to a cell testing device, a cell testing method, a program, and a recording medium.

Priority is claimed on Japanese Patent Application No. 2018-005648, filed January 17, 2018, the content of which is incorporated herein by reference.

### [Background Art]

In cell culture, the linearity of the number of viable cells and capacitance is strong in a first half of the culture in which a survival rate is high. Thus, a technique of measuring the number of viable cells within a vessel during culture using an impedance sensor is generally applicable to cell culture in a liquid. However, in this technique, there is linearity in the first half of the culture in which the survival rate is maintained at a high level, but the linearity is lost in a second half of the culture in which the survival rate is reduced and an estimated value may deviate from an actual measured value based on sampling.

A technique of processing data acquired by an impedance sensor with software on a computer to correct the data and obtain an estimated value with respect to the deviation between the estimated value and the actual measured value based on sampling has been proposed (for example, Patent Literature 1).

In the technology described in Patent Literature 1, a viable cell volume during culture is used as correct answer data. The viable cell volume is measured by flow cytometry. In culture data used in the technology described in Patent Literature 1, a viable cell volume and an estimated value from acquired capacitance data for each frequency match in the first half of the culture, but deviate in the second half of the culture.

Thus, in the technology described in Patent Literature 1, data acquired by the impedance sensor is divided into capacitance data for each frequency and further normalized using a maximum value and a minimum value. In the technology described in Patent Literature 1, a culture period is divided into a first half and a second half at a frequency based on an amount of deviation, the horizontal axis represents a frequency, the vertical axis represents a normalized value, and a corrected value for the viable cell volume is obtained from a ratio between an integrated area of the first half and an integrated area of the second half. The technology described in Patent Literature 1 is applied to cell culture having a correlation between an amount of deviation between a measured value and an estimated value and the above-described area ratio using data acquired by the impedance sensor.

### [Citation List]

### [Patent Literatures]

[Patent Literature 1] United States Patent No. 9568449

### [Summary of Invention]

### [Technical Problem]

However, in the technology described in Patent Literature 1, the estimation of the viable cell volume may be limited to specific culture conditions.

An aspect of the present invention provides a cell testing device, a cell testing method, a program, and a recording medium capable of implementing viable cell count estimation with high accuracy during a prescribed period within a culture period from seeding in cell culture to death at a certain survival rate or less.

### [Solution to Problem]

A cell testing device (2) according to one aspect of the present invention includes: an impedance sensor (a sensor 20, a probe 21, or a sensor unit 22) configured to measure impedance of a culture solution; a storage unit (27) configured to store a coefficient for estimating the number of viable cells present in the culture solution during a prescribed period using the impedance for each of a plurality of periods into which the prescribed period within a culture period from the start of cell culture to death is divided; and a viable cell count estimation unit (a control unit 24, a calculation unit 26 or a viable cell count estimation unit 28) configured to acquire the impedance and estimate the number of viable cells using at least one of the acquired impedance and a coefficient for each period stored in the storage unit.

In the cell testing device according to one aspect of the present invention, the storage unit may store the coefficient for each of a plurality of periods into which the prescribed period is divided based on the impedance measured by the impedance sensor, information about the number of viable cells, and an impedance measurement time.

In the cell testing device according to one aspect of the present invention, the viable cell count estimation unit may perform principal component analysis with respect to the impedance, the information about the number of viable cells, and an elapsed time from a start time of the prescribed period in case that the impedance and the information about the number of viable cells are measured during a period from the start of the cell culture to the death, the viable cell count estimation unit may divide the prescribed period into a plurality of periods based on a result of performing the principal component analysis, the viable cell count estimation unit may obtain the coefficient for each of the periods into which the prescribed period is divided based on the result of performing the principal component analysis, and the viable cell count estimation unit may cause the storage unit to store the coefficient obtained for each of the periods into which the prescribed period is divided.

In the cell testing device according to one aspect of the present invention, the viable cell count estimation unit may obtain the coefficient using at least one of linear regression, partial least squares regression, and quadratic or higher regression.

In the cell testing device according to one aspect of the present invention, the viable cell count estimation unit may increase the elapsed time for each prescribed period, obtains a ratio difference or a ratio between a change in the impedance and a change in the number of viable cells, obtain a branch point for an n^{th} (n is an integer of 1 or more) period and an (n+1)^{th} period in case that the difference or the ratio is outside of a prescribed range, and classify the n^{th} period based on the obtained branch point.

In the cell testing device according to one aspect of the present invention, the viable cell count estimation unit may obtain boundary information including a branch point for the plurality of periods and capacitance of the impedance at the branch point and causes the storage unit to store the obtained boundary information.

In the cell testing device according to one aspect of the present invention, the viable cell count estimation unit may determine a period to which capacitance of the acquired impedance corresponds based on the boundary information stored in the storage unit, and the viable cell count estimation unit may estimate the number of viable cells using the acquired impedance and the coefficient associated with the determined period.

A cell testing method, according to one aspect of the present invention, for use in a cell testing device including an impedance sensor, a storage unit configured to store a coefficient for estimating the number of viable cells present in a culture solution during a prescribed period using impedance measured by the impedance sensor for each of a plurality of periods into which the prescribed period within a culture period from the start of cell culture to death is divided, and a viable cell count estimation unit, includes: measuring, by the impedance sensor, the impedance of the culture solution during the prescribed period; and acquiring, by the viable cell count estimation unit, the impedance and estimating the number of viable cells using at least one of the acquired impedance and the coefficient for each period stored in the storage unit.

A program, according to one aspect of the present invention, causes a computer of a cell testing device including an impedance sensor, a storage unit configured to store a coefficient for estimating the number of viable cells present in a culture solution during a prescribed period using impedance measured by the impedance sensor for each of a plurality of periods into which the prescribed period within a culture period from the start of cell culture to death is divided, and a viable cell count estimation unit to: acquire the impedance of the culture solution measured by the impedance sensor; and estimate the number of viable cells using at least one of the acquired impedance and the coefficient for each period stored in the storage unit.

A computer-readable recording medium, according to one aspect of the present invention, recording the above-described program.

### [Advantageous Effects of Invention]

According to an aspect of the present invention, it is possible to implement viable cell count estimation with high accuracy during a prescribed period within a culture period from seeding in cell culture to death at a certain survival rate or less.

### [Brief Description of Drawings]

Fig. 1 is a diagram showing a configuration example of a cell testing system according to the present embodiment.
Fig. 2 is a diagram showing an example of results of principal component analysis of capacitance and a viable cell density according to the present embodiment.
Fig. 3 is an example in which the results of the principal component analysis shown in Fig. 2 are classified into three phases.
Fig. 4A is a diagram showing results of principal component analysis in a first phase I of Fig. 2.
Fig. 4B is a diagram showing results of principal component analysis in a second phase II of Fig. 2.
Fig. 4C is a diagram showing results of principal component analysis in a third phase III of Fig. 2.
Fig. 5 is a flowchart showing an example of a processing procedure to be performed by a viable cell count estimation unit in a learning operation mode according to the present embodiment.
Fig. 6 is a diagram showing an example of information stored in a storage unit according to the embodiment.
Fig. 7 is a flowchart showing an example of a processing procedure to be performed by the viable cell count estimation unit in an estimation operation mode according to the present embodiment.
Fig. 8 is a diagram showing an example of a result of estimating the number of viable cells after learning according to the present embodiment.
Fig. 9 is a diagram showing an example of the number of viable cells estimated during the entire culture period and the number of viable cells actually measured offline according to the present embodiment.
Fig. 10 is a diagram showing an example in which an estimated value deviates from an actual measured value according to a comparative example.

### [Description of Embodiments]

Hereinafter, embodiments of the present invention will be described with reference to the drawings.

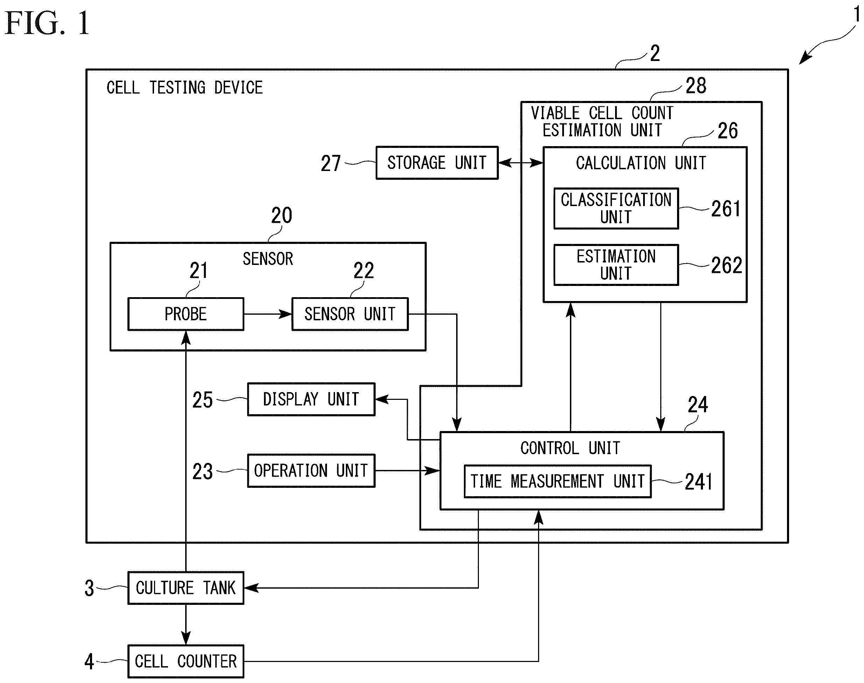

Fig. 1 is a diagram showing a configuration example of a cell testing system 1 according to the present embodiment. As shown in Fig. 1, the cell testing system 1 includes a cell testing device 2, a culture tank 3, and a cell counter 4. The cell testing device 2 includes a sensor 20, an operation unit 23, a display unit 25, a storage unit 27, and a viable cell count estimation unit 28. The viable cell count estimation unit 28 includes a control unit 24 and a calculation unit 26. The sensor 20 includes a probe 21 and a sensor unit 22. The control unit 24 includes a time measurement unit 241. The calculation unit 26 includes a classification unit 261 and an estimation unit 262.

The sensor 20, the operation unit 23, and the display unit 25 are connected to the control unit 24 of the viable cell count estimation unit 28. The control unit 24 is connected to the calculation unit 26. The storage unit 27 is connected to the calculation unit 26. The cell counter 4 outputs an acquired measured value to the control unit 24. The cell counter 4 and the control unit 24 may be connected by wire or wirelessly.

The culture tank 3 contains a culture solution containing cells. The sensor 20, a stirrer (not shown), a heater (not shown), and the like are attached to the culture tank 3 as in Patent Literature 1. The stirrer and the heater are controlled by the cell testing device 2. The cells to be tested are, for example, Chinese hamster ovary (CHO) cells.

The cell counter 4 samples the culture solution, stains the viable cells, and measures the viable cell density (VCD) [cells/mL] in the culture solution. A timing at which the sensor 20 measures the capacitance does not need to coincide with a timing at which the viable cell density is acquired. The measurement timing may be given so that the capacitance is continuously measured and the viable cell density is intermittently acquired. The cell counter 4 outputs information indicating the measured viable cell density to the cell testing device 2. The measurement by the cell counter 4 is performed offline and used in the learning operation mode. The learning operation mode is an operation mode in which estimation target cells are used and cultured in advance and information during the culture is learned so that the number of viable cells is estimated. During the culture, the distribution of cells in the culture solution is assumed to be uniform because the culture solution is stirred by the stirrer. The "viable cell density" is an example of the "number of viable cells". Hereinafter, the "viable cell density" may be referred to as the "number of viable cells".

In the learning operation mode, the cell testing device 2 acquires information indicating the viable cell density measured by the cell counter 4 and a measured value measured by the sensor 20. The cell testing device 2 starts the measurement of the time at which the culture has started, measures the time at which the information indicating the viable cell density and the measured value have been acquired, and stores the measured time (elapsed time) in association with the acquired information indicating the viable cell density and the measured value. The cell testing device 2 performs preprocessing for estimating the number of cells based on the stored information. The preprocessing for estimating the number of cells will be described below.

The cell testing device 2 acquires the measured value measured by the sensor 20 in the estimation operation mode in which the estimation is performed using learned data. The cell testing device 2 starts the measurement of the time at which the culture has started and measures the time at which the measured value has been acquired. The cell testing device 2 estimates the number of viable cells in the culture solution based on the preprocessing for estimating the number of cells and the measured value.

The probe 21 includes a measurement probe and an impedance change sensor. The impedance change sensor is a sensor that measures the impedance of a culture solution containing cells. A measured value measured by the impedance change sensor includes impedance for each of a plurality of different frequencies in a prescribed frequency range (for example, a range of 10 kHz to 100 MHz). The probe 21 measures the impedance of the culture solution and outputs a measured value to the sensor unit 22. The probe 21 may include an amplification unit that amplifies the measured value.

The sensor unit 22 separates the measured value output by the probe 21 into two components of capacitance (dielectric permittivity) and dielectric conductance (conductivity) in a known technique and outputs the capacitance and the conductance into which the measured value is separated to the control unit 24. The sensor unit 22 may include an amplification unit that amplifies the measured value measured by the probe. The capacitance includes, for example, a frequency component of 10 kHz to 100 MHz.

The operation unit 23 is, for example, a touch panel sensor, an operation button, or the like provided on the display unit 25. The operation unit 23 detects an operation result of a user operation and outputs the detected operation result to the control unit 24. The operation result includes a cell name, set values of culture conditions, an operation mode, a culture start instruction, a culture end instruction, and the like. The operation mode includes a learning operation mode and an estimation operation mode.

The control unit 24 outputs information indicating the learning operation mode or information indicating the estimation operation mode to the calculation unit 26 based on the operation result output by the operation unit 23. The control unit 24 starts the culture by controlling the stirrer, the heater, and the like of the culture tank 3 according to the set values of the culture conditions in accordance with the culture start instruction. The control unit 24 starts time measurement using the time measurement unit 241 at the start of culture. In the learning operation mode and the estimation operation mode, the control unit 24 acquires a measured value output by the sensor unit 22 at prescribed time intervals in an online process. In the learning operation mode and the estimation operation mode, the control unit 24 outputs information indicating the elapsed time measured in case that the measured value has been acquired to the calculation unit 26 in association with the measured value. The control unit 24 may be configured to cause the storage unit 27 to store the number of viable cells, the measured value, and the elapsed time. In the learning operation mode, the control unit 24 acquires information indicating the viable cell density output by the cell counter 4 at each timing in case that the measured value of the sensor unit 22 is acquired in the offline process. In the learning operation mode, the control unit 24 outputs information indicating the elapsed time measured in case that the viable cell density has been acquired to the calculation unit 26 in association with the viable cell density. The control unit 24 may cause the storage unit 27 to store the number of viable cells and the elapsed time. The control unit 24 acquires information indicating the estimated number of viable cells output by the calculation unit 26 in the estimation operation mode. The information indicating the estimated number of viable cells output by the calculation unit 26 includes the elapsed time from the start of culture. The control unit 24 generates an image of a result of estimating the number of cells based on the information indicating the estimated number of viable cells output by the calculation unit 26 and causes the generated image of the result of estimating the number of cells to be displayed on the display unit 25.

The display unit 25 is, for example, a liquid crystal display device, an organic electro luminescence (EL) display device, or the like. The display unit 25 displays the display image output by the control unit 24. The display image is a culture condition setting screen, the image of the result of estimating the number of cells, or the like.

In the learning operation mode, the classification unit 261 of the calculation unit 26 acquires a period from the start of culture to the end of culture, the measured value output by the control unit 24, and information indicating the elapsed time measured in case that the measured value has been acquired, in real time. In the learning operation mode, the classification unit 261 acquires information indicating the viable cell density in the off-time and information indicating the elapsed time measured in case that the information indicating the viable cell density has been acquired in an offline process. In the learning operation mode, the classification unit 261 causes the storage unit 27 to store the viable cell density and information indicating the elapsed time measured in case that the measured value and the viable cell density have been acquired in association with the acquired period from the start of culture to the end of culture and the measured value. In the learning operation mode, the classification unit 261 performs the principal component analysis using the stored measured values of the period from the start of culture to the end of culture and the information indicating the viable cell density and the elapsed time. The classification unit 261 uses a capacitance value among the measured values. Thus, as an example, if the frequency range includes 18 points in the range of 287 kHz to 20 MHz, the number of items of data used for the principal component analysis of the classification unit 261 is 18 (frequencies) × 1 batch. One batch is a period from the start of culture to the end of culture. In the learning operation mode, the classification unit 261 obtains boundary information for classifying the measured value and the number of viable cells based on results of the principal component analysis. In the learning operation mode, the classification unit 261 classifies the results of the principal component analysis based on the obtained boundary information and obtains information indicating the measured value and the viable cell density included in each classified phase. In the learning operation mode, the classification unit 261 causes the storage unit 27 to store the obtained boundary information and the information indicating the measured value and the viable cell density included in each phase. In the learning operation mode, the estimation unit 262 of the calculation unit 26 obtains a coefficient for each classified phase in which the relationship between the principal component of the capacitance and the viable cell density is classified, for example, according to linear regression, partial least squares regression (PLS), or quadratic or higher regression, based on the obtained boundary information and causes the storage unit 27 to store the obtained coefficient.

In the estimation operation mode, the estimation unit 262 of the calculation unit 26 acquires information indicating a period from the start of culture to the end of culture, a measured value output by the control unit 24, and an elapsed time measured in case that the measured value has been acquired. In the estimation operation mode, the estimation unit 262 determines a phase to which the acquired capacitance corresponds using boundary information stored by the storage unit 27 with respect to a capacitance value among the measured values. The phase will be described below. In the estimation operation mode, the estimation unit 262 estimates the number of viable cells by correcting the measured capacitance using a coefficient in accordance with a determination result. The estimation unit 262 outputs the information indicating the elapsed time measured in case that the measured value has been acquired to the control unit 24 in association with information indicating the estimated number of viable cells.

The storage unit 27 stores cell names, set values of culture conditions, and the like. The storage unit 27 stores the measured values, the viable cell density, the elapsed time, and the like in the learning operation mode. The storage unit 27 stores the boundary information determined in the learning operation mode, the coefficient of each boundary, and the like. The storage unit 27 stores a threshold value of a separation probability r used in case that the phase is classified. The separation probability r will be described below.

### <Learning operation mode>

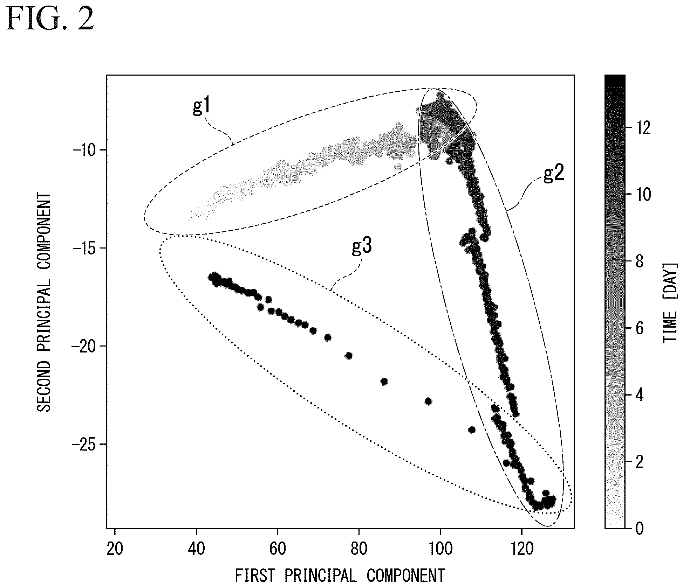

Next, an example of a process to be performed by the classification unit 261 and the estimation unit 262 in the learning operation mode will be described with reference to Figs. 2 to 4C. It is assumed that a proportion of the measured value of the capacitance for each frequency and a relationship between variables in the viable cell density and the capacitance change at the same time and are steady in case that there is no change. That is, in case that a curve of the capacitance is within a certain range, the relationship between the certain viable cell density and the capacitance is established, so that detection can be performed in a single technique. In case that the relationship between the viable cell density and the capacitance has changed, it is assumed that the proportion of the capacitance for each frequency changes and the change can be detected. Under this assumption, it is possible to divide the entire culture period for each phase where the state of the capacitance is steady and to determine an estimation technique and a coefficient for each phase of the division.

Fig. 2 is a diagram showing an example of results of principal component analysis of capacitance and a viable cell density according to the present embodiment. In Fig. 2, the horizontal axis represents a first principal component and the vertical axis represents a second principal component. The shading at each point indicates an elapsed time in the culture and indicates that the lighter the color is, the shorter the elapsed time is, i.e., the first half of the culture. The shading at each point indicates that the darker the color is, the longer the elapsed time is, i.e., the second half of the culture.

As shown in Fig. 2, the result of the principal component analysis can be regarded to be a triangle surrounded by substantially straight lines denoted by reference signs g1 to g3. In the case of such a principal component analysis result, it is possible to mathematically determine one of the sides of the reference signs g1 to g3 to which the data point belongs.

In the learning operation mode, the classification unit 261 divides the results of the principal component analysis into three phases using, for example, logistic regression. The classification unit 261 provides capacitance data and correct answer labels "first phase I", "second phase II", and "third phase III" for each time. Furthermore, the classification unit 261 obtains a boundary vector between the labels according to regression. Data indicated by a substantially straight line indicated by the reference sign g1 is data of the first phase I. Data indicated by a substantially straight line indicated by the reference sign g2 is data of the second phase II. Data indicated by a substantially straight line indicated by the reference sign g3 is data of the third phase III.

The first phase I, the second phase II, and an example of a determination technique will be described. In the following description, the start time of the culture is set to 0 and the elapsed time is set to t. The classification unit 261 obtains a ratio between a change in the viable cell density and a change in the capacitance from time 0 to time t [{viable cell density (t) - viable cell density (0)}/{ capacitance (t) - capacitance (0)}]. In case that the measurement interval is, for example, 1 hour, the classification unit 261 increases time t every hour and sequentially obtains ratios. That is, the classification unit 261 calculates a ratio after the elapsed time of one hour, a ratio after the elapsed time of two hours, a ratio after the elapsed time of three hours, and the like. The classification unit 261 calculates a difference or a ratio between the ratio in case that the elapsed time is one hour and the ratio in case that the elapsed time is two hours, and determines whether the difference is within a prescribed range or whether the ratio is within a prescribed range. In case that the difference or the ratio is within the prescribed range, the classification unit 261 determines that the linearity is continuous. In case that the difference or the ratio is outside of the prescribed range, the classification unit 261 determines that the linearity is not continuous and determines that there is a branch point between the first phase I and the second phase II.

The classification unit 261 sets this branch point as the start point of the second phase II and calculates a ratio between a change in the number of viable cells from the start point (the branch point) to time t and a change in the second component in case that the principal component analysis has been performed. The classification unit 261 calculates a difference or a ratio between the ratio in case that the elapsed time is one hour and the ratio in case that the elapsed time is two hours and determines whether or not the difference is within a prescribed range or whether or not the ratio is within a prescribed range. In case that the difference or the ratio is outside of the prescribed range, the classification unit 261 determines that the linearity is not continuous and determines that there is a branch point between the second phase II and the third phase III. The branch point calculation technique described above is an example and the present invention is not limited thereto. In case that there are four or more phases, the classification unit 261 further obtains a branch point between the n^{th} phase and the (n+1)^{th} phase.

The boundary information will be described.

The boundary information includes the above-described branch point and the capacitance included in the branch point. The classification unit 261 obtains the capacitance included in the branch point to determine a phase. The classification unit 261 calculates a coefficient for each phase. In case that there are three phases, the coefficients of the phases are a first coefficient used in the first phase I, a second coefficient used in the second phase II, and a third coefficient used in the third phase. The classification unit 261 classifies the phases using a separation probability r. The classification unit 261 calculates the separation probability r for each item of data from the coefficient and the capacitance. For example, in the case of a classification into two phases, the classification unit 261 determines that the phase is the first phase I in case that the separation probability r is less than 0.5 and determines that the phase is the second phase II in case that the separation probability r is greater than or equal to 0.5 in a process of determining the first phase I and the second phase II.

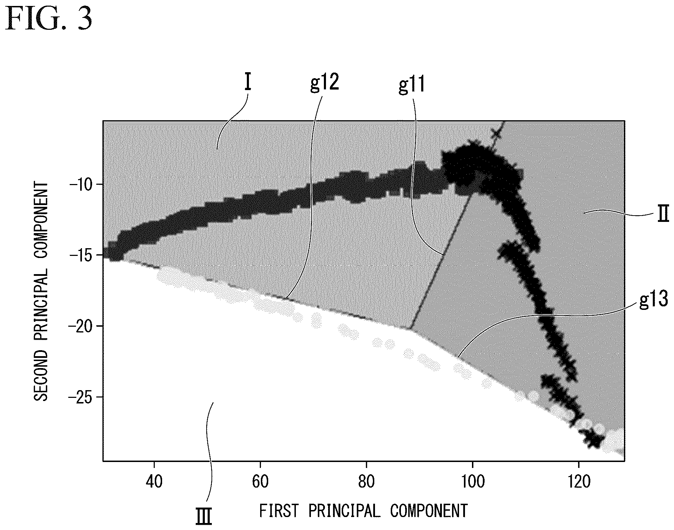

Fig. 3 shows results of separating the phases using the coefficient for determination.

In case that the data points shown in Fig. 2 are separated into phases based on reference signs g1 to g3, the separation results are as shown in Fig. 3.

Fig. 3 is an example in which the results of the principal component analysis shown in Fig. 2 are classified into three phases. In Fig. 3, the horizontal axis represents a first principal component and the vertical axis represents a second principal component.

In the example shown in Fig. 3, the data points are classified into three phases of a first phase I, a second phase II, and a third phase III by line segments g11, g12, and g13.

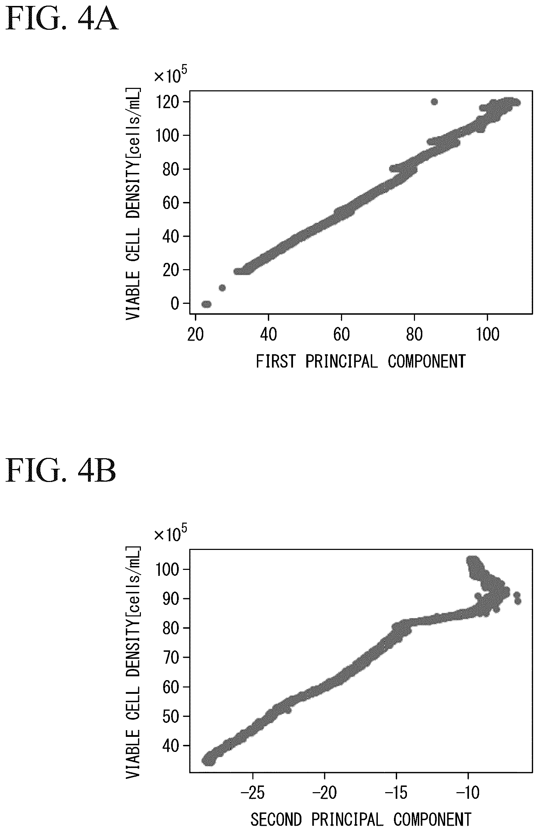

Next, the classification unit 261 checks relationships between the phases after the division and the viable cell density and the capacitance during the culture period in each of the phases. The relationship between the principal component and the viable cell density due to the capacitance in each of the three phases obtained through the division as shown in Fig. 3 is as shown in Figs. 4A to 4C.

Figs. 4A to 4C are diagrams in which the results of the principal component analysis of Fig. 2 are divided into three phases. Fig. 4A shows results of the principal component analysis in the first phase I. The horizontal axis represents a first principal component and the vertical axis represents a viable cell density [cells/mL].

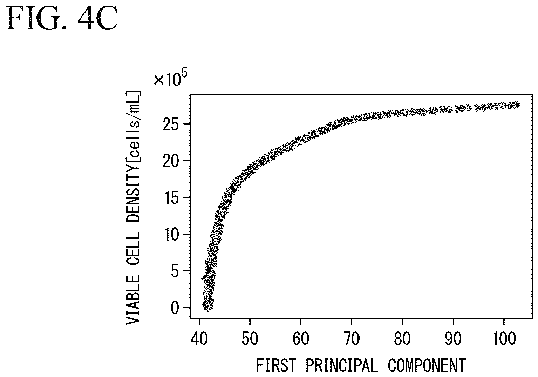

Fig. 4B shows results of the principal component analysis in the second phase II. The horizontal axis represents a second principal component and the vertical axis represents a viable cell density [cells/mL]. Fig. 4C shows results of the principal component analysis in the third phase III. The horizontal axis represents a first principal component and the vertical axis represents a viable cell density [cells/mL].

The estimation unit 262 obtains the relationship between the capacitance and the number of viable cells according to, for example, linear regression or partial least squares regression on the first principal component, with respect to Fig. 4A. The estimation unit 262 obtains the relationship between the capacitance and the number of viable cells according to, for example, linear regression or partial least squares regression on the second principal component, with respect to Fig. 4B. The estimation unit 262 obtains the relationship between the capacitance and the number of viable cells according to, for example, quadratic or higher regression on the first principal component, with respect to Fig. 4C. The estimation unit 262 causes the storage unit 27 to store the obtained coefficient in association with the phase.

Here, the principal component is the frequency component of the capacitance. For example, in case that the frequency range of the capacitance is 287 kHz to 20 MHz, the frequency of the first principal component is 600 kHz and the frequency of the second principal component is 287 kHz to 20 MHz.

The estimation unit 262 obtains the coefficient of the first phase I according to linear regression using a first coefficient for distinguishing between the first phase I and the second phase II and the capacitance and the viable cell density of, for example, 600 kHz.

The estimation unit 262 obtains the coefficient of the second phase II according to partial least squares regression using a second coefficient for distinguishing between the second phase II and the third phase III and the capacitance and the viable cell density of, for example, 287 kHz to 20 MHz.

Further, the estimation unit 262 obtains the coefficient of the third phase III according to quadratic regression using the second coefficient for distinguishing between the second phase II and the third phase III and the capacitance and the viable cell density of, for example, 600 kHz.

The above-described method of obtaining the coefficient is an example and the present invention is not limited thereto.

Although an example in which the results of the principal component analysis are divided into three types is shown in the examples shown in Figs. 2 to 4C, the present invention is not limited thereto. The number of divisions may be two or four or more based on the results obtained by the principal component analysis. The estimation unit 262 may be configured to obtain the coefficient for each phase obtained through the division.

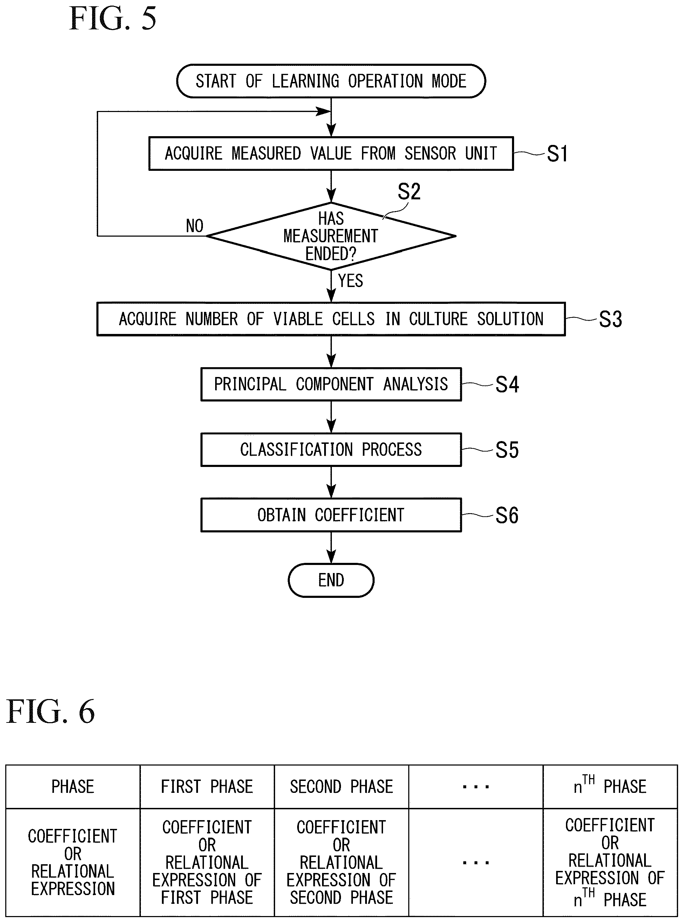

Fig. 5 is a flowchart showing an example of a processing procedure to be performed by the viable cell count estimation unit 28 in the learning operation mode according to the present embodiment. Fig. 5 is an example in which the results of the principal component analysis are classified into n (n is an integer of 2 or more) types.
(Step S1) The control unit 24 acquires a measured value output from the sensor unit 22 in an online process and measures an elapsed time from the start of culture in case that the measured value has been acquired. Subsequently, the control unit 24 outputs information indicating the measured value and the elapsed time to the calculation unit 26. Subsequently, the classification unit 261 acquires the information indicating the measured value and the elapsed time output by the control unit 24. Subsequently, the classification unit 261 causes the storage unit 27 to store the acquired information indicating the measured value and the elapsed time.
(Step S2) The control unit 24 determines whether or not the measurement has ended. The measurement is ended, for example, by the control unit 24 detecting that the operation unit 23 has been operated by an operator to end the measurement. In case that it is determined that the measurement has not ended (step S2; NO), the control unit 24 returns the process to step S1. In case that it is determined that the measurement has ended (step S2; YES), the control unit 24 moves the process to step S3.
(Step S3) The control unit 24 acquires information indicating a viable cell density in a culture solution from the start of culture to the end of culture at each timing measured by the sensor 20 in an offline process and measures an elapsed time in case that the viable cell density has been acquired. Subsequently, the control unit 24 outputs information indicating the viable cell density and the elapsed time to the classification unit 261. Subsequently, the classification unit 261 acquires the information indicating the viable cell density and the elapsed time output by the control unit 24 and causes the storage unit 27 to store the acquired information indicating the viable cell density and the elapsed time.
(Step S4) The classification unit 261 performs principal component analysis using measured values, the viable cell density, and the elapsed time during a period from the start of culture to the end of culture stored in the storage unit 27.
(Step S5) The classification unit 261 obtains boundary information for classifying the measured values and the viable cell density using, for example, logistic regression, based on results of the principal component analysis. Subsequently, the classification unit 261 classifies the results of the principal component analysis based on the obtained boundary information and obtains information indicating the measured value and the viable cell density included for each classified phase.
(Step S6) The estimation unit 262 obtains a coefficient for each phase in which the relationship between the principal component of the capacitance and the viable cell density is classified, for example, according to linear regression, partial least squares regression, or quadratic or higher regression, based on the obtained boundary information. Subsequently, the estimation unit 262 causes the storage unit 27 to store the obtained coefficient.

Thus, the learning operation mode ends.

Although the example in which the viable cell density is acquired in the offline process has been described in the above-described example, the viable cell density may be acquired in the online process. The cell testing device 2 may perform the above-described learning for each cell or culture condition. In this case, the cell testing device 2 may cause the storage unit 27 to store the boundary information and the coefficient in association for each cell or culture condition.

Although an example in which a coefficient is obtained for each phase has been described in the above-described example, a relational expression may be used.

Although an example in which the viable cell density is measured by the cell sensor in the learning operation mode has been described in the above-described example, the present invention is not limited thereto. The viable cell density or the number of viable cells may be measured by another sensor or a measurement instrument.

Although an example in which the period from the start of cell culture to death is classified into a plurality of phases has been described with reference to in Figs. 2 to 5, the present invention is not limited thereto. The classification unit 261 may be configured to perform principal component analysis for any period in the cell culture period and perform a classification into phases. In this case, the estimation unit 262 also may obtain a coefficient for each classified phase for any period in the cell culture period.

Although an example in which the principal component analysis is performed after the measurement ends and the coefficient is obtained for each classified phase (period) has been described with reference to Fig. 5, the present invention is not limited thereto. The classification unit 261 may be configured to estimate a coefficient while performing the principal component analysis, for example, at each timing in case that the measured value has been acquired, from a start time of culture to an elapsed time or from a start time of a prescribed period to an elapsed time with respect to the measured values acquired from the sensor unit 22 and the cell counter 4. In case that the measured value is switched to a different phase, the classification unit 261 causes the storage unit 27 to store the final coefficient of the phase before the switching and estimates a coefficient of a new phase after initializing the coefficient. That is, the classification unit 261 may be configured to perform an online classification of the phase and estimation of the coefficient.

Further, the estimation unit 262 may estimate the number of viable cells using phases classified online as described above and estimated coefficients.

Although an example in which the number of viable cells is estimated using one of the coefficients set for each phase has been described in the above-described example, the present invention is not limited thereto. The estimation unit 262 may be configured to estimate the number of viable cells using a coefficient obtained by multiplying the coefficient of the first phase by 60% and multiplying the coefficient of the second phase by 40%, for example, in switching between the first phase I and the second phase II. Thereby, according to the present embodiment, it is possible to continuously switch the phase in switching between the phases.

An example of information stored in the storage unit 27 according to the learning operation mode will be described.

Fig. 6 is a diagram showing an example of information stored in the storage unit 27 according to the present embodiment. As shown in Fig. 6, the storage unit 27 stores a coefficient for estimating the number of viable cells for each phase.

### <Estimation operation mode>

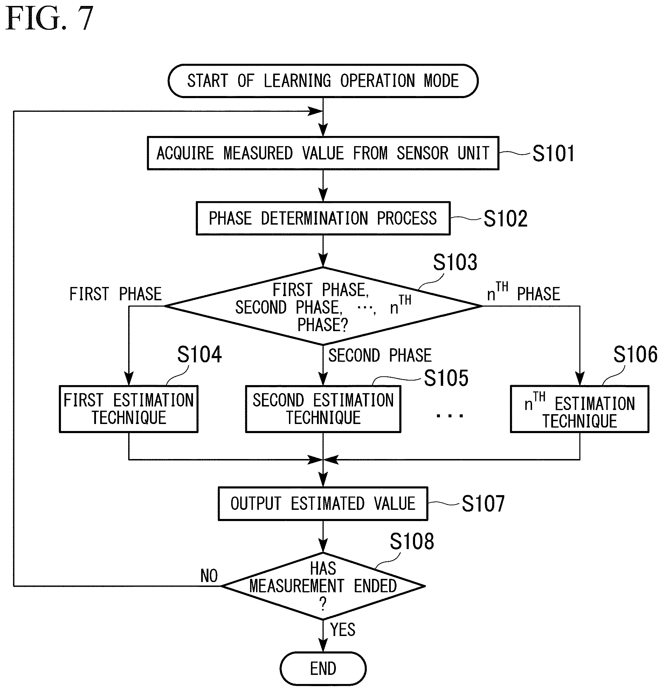

Next, an example of a process to be performed by the estimation unit 262 in the estimation operation mode will be described with reference to Fig. 7. Fig. 7 is a flowchart showing an example of a processing procedure to be performed by the viable cell count estimation unit 28 in the estimation operation mode according to the present embodiment. Fig. 7 shows an example in which the results of the principal component analysis in the learning operation mode are classified into n (n is an integer of 2 or more) types.
(Step S101) The control unit 24 acquires a measured value output by the sensor unit 22 in an online process and measures an elapsed time from the start of the culture in case that the measured value has been acquired. Subsequently, the control unit 24 outputs information indicating the measured value and the elapsed time to the calculation unit 26. Subsequently, the classification unit 261 acquires the information indicating the measured value and the elapsed time output by the control unit 24. The classification unit 261 may cause the storage unit 27 to store the acquired information indicating the measured value and the elapsed time.
(Step S102) The estimation unit 262 determines a phase to which the acquired capacitance corresponds using boundary information stored in the storage unit 27 with respect to a capacitance value of measured values.
(Step S103) As a result of the determination, the estimation unit 262 determines whether the capacitance belongs to a first phase, a second phase, ..., an n^{th} phase. In case that the estimation unit 262 determines that the capacitance belongs to the first phase (step S103; first phase), the process proceeds to step S104. In case that the estimation unit 262 determines that the capacitance belongs to the second phase (step S104; second phase), the process proceeds to step S105. In case that the estimation unit 262 determines that the capacitance belongs to the n^{th} phase (step S103; n^{th} phase), the process proceeds to step S106.
(Step S104) The estimation unit 262 estimates the number of viable cells by correcting the measured value using the coefficient of the first phase stored in the storage unit 27. For example, the estimation unit 262 obtains an estimated value of the number of viable cells by multiplying the capacitance of the measured value by the coefficient. In the present embodiment, estimating the number of viable cells by correcting the measured value using the coefficient of the first phase stored in the storage unit 27 as described above is referred to as a first estimation technique. After the processing, the estimation unit 262 moves the process to step S107.
(Step S105) The estimation unit 262 estimates the number of viable cells by correcting the measured value using the coefficient of the second phase stored in the storage unit 27. In the present embodiment, estimating the number of viable cells by correcting the measured value using the coefficient of the second phase stored in the storage unit 27 as described above is referred to as a second estimation technique. After the processing, the estimation unit 262 moves the process to step S107.
(Step S106) The estimation unit 262 estimates the number of viable cells by correcting the measured value using the coefficient of the n^{th} phase stored in the storage unit 27. In the present embodiment, estimating the number of viable cells by correcting the measured value using the coefficient of the n^{th} phase stored in the storage unit 27 as described above is referred to as an n^{th} estimation technique. After the processing, the estimation unit 262 moves the process to step S107.
(Step S107) The estimation unit 262 associates information indicating the estimated number of viable cells with information indicating the elapsed time and outputs the information to the control unit 24. Subsequently, the control unit 24 generates image information using the information indicating the estimated number of viable cells and the information indicating the elapsed time output by the estimation unit 262 and causes the display unit 25 to display the generated image information.
(Step S108) The control unit 24 determines whether or not the measurement has ended. The measurement is ended, for example, by the control unit 24 detecting that the operation unit 23 has been operated by the operator to end the measurement. In case that it is determined that the measurement has not ended (step S108; NO), the control unit 24 returns the process to step S101. In case that it is determined that the measurement has ended (step S108; YES), the control unit 24 ends the process.

The estimation unit 262 may estimate the number of viable cells using a coefficient for each classified phase for any period in the cell culture period.

The capacitance used for estimation may use data of a frequency range or a frequency suitable for the phase. As the frequency range or frequency to be used, a range or frequency having the best balance between linearity and stability among measurement frequencies may be selected.

Next, an example of estimation results after learning as described above will be described.

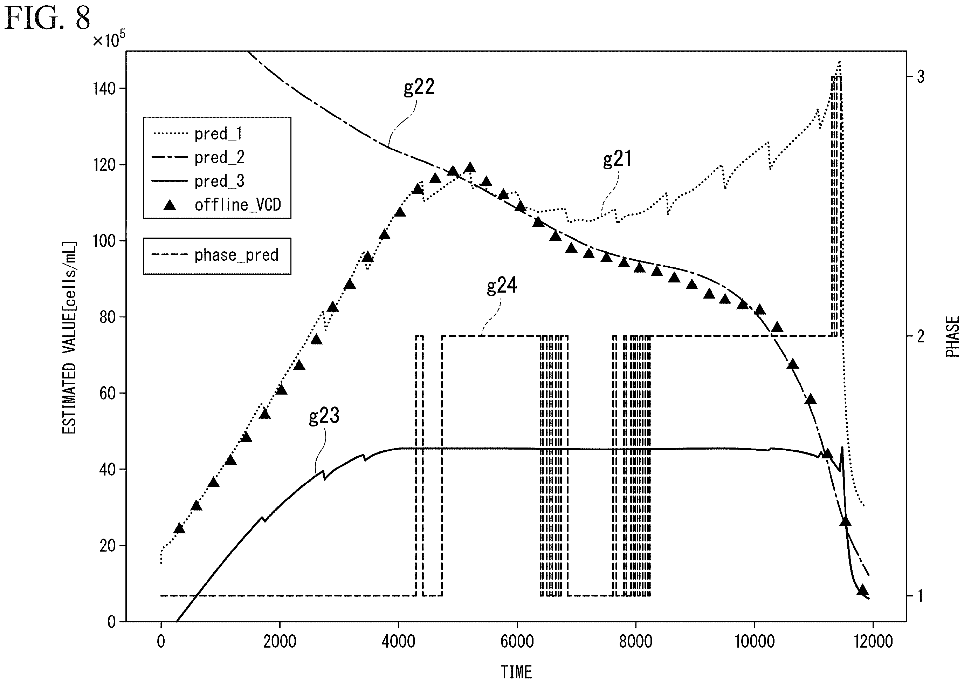

Fig. 8 is a diagram showing an example of a result of estimating the number of viable cells after learning according to the present embodiment. In Fig. 8, the horizontal axis represents time and the left vertical axis represents an estimated value [cells/mL] of a viable cell density. A numerical value of the time on the horizontal axis is the number of acquired data points, which is the elapsed time from the start of the measurement. In case that the numerical value is converted in units of date and time, 2000 [points] ≈ 56 [hours]. 1 on the right vertical axis indicates that it belongs to the first phase, 2 on the right vertical axis indicates that it belongs to the second phase, and 3 on the right vertical axis indicates that it belongs to the third phase.

A triangle mark indicates a measured viable cell density. A dashed line g21 indicates a viable cell density estimated in the first estimation technique. A dashed-dotted line g22 indicates a viable cell density estimated in the second estimation technique. A solid line g23 indicates a viable cell density estimated in the third estimation technique. A dashed line g24 indicates a classified phase.

Although there is a period in which the result of distinguishing between the first phase and the second phase and the result of distinguishing between the second phase and the third phase change in two phases as indicated by a reference sign g24 of Fig. 8, an influence on the estimation result is small regardless of which phase is determined because the estimated values are close to each other.

Next, an example of the number of viable cells estimated in the entire culture period by applying the present embodiment will be described.

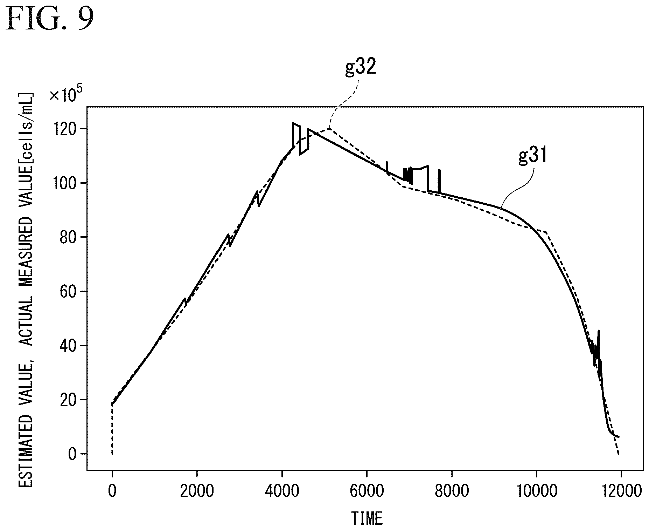

Fig. 9 is a diagram showing an example of the number of viable cells estimated during the entire culture period and the number of viable cells actually measured offline according to the present embodiment. In Fig. 9, the horizontal axis represents time and the vertical axis represents an actual measured value and an estimated value of a viable cell density [cells/mL]. A numerical value of the time on the horizontal axis is the number of acquired data points, which is the elapsed time from the start of measurement. In case that the numerical value is converted in units of date and time, 2000 [points] ≈ 56 [hours]. A reference sign g31 indicates an estimated value and a reference sign g32 indicates an actual measured value.

Although there are also points where the estimated values are discontinuous in Fig. 9, they are points where the phase is switched as in Fig. 8. If the actual measured value is used as the correct answer data as shown in Fig. 9, it is possible to accurately estimate the number of viable cells across the entire period in the present embodiment.

A comparative example in which the estimated value and the measured value deviate will be described.

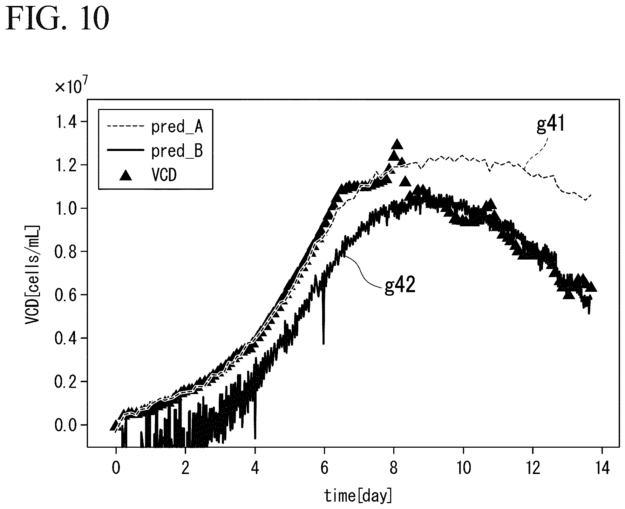

In the technique of the related art described in Patent Literature 1, the deviation in the first half of the culture may increase while the deviation in the second half of the culture decreases. On the other hand, in the comparative example, a correction formula of the technique of the related art described in Patent Literature 1 is modified to minimize an amount of correction in the first half of the culture and secure an amount of correction in the second half of the culture. Although there is also a culture state in which correction can be appropriately performed in the case of the above-described correction, a deviation may occur between the estimated value and the actual measured value in the first half of the culture as shown in Fig. 10.

Fig. 10 is a diagram showing an example in which the estimated value and the measured value deviate according to the comparative example. In Fig. 10, the horizontal axis represents elapsed time and the vertical axis represents a viable cell density (VCD). A triangle mark indicates an actual measured value of the viable cell density. A reference sign g41 indicates an estimated value in the first half of the culture according to the comparative example. A reference sign g42 indicates an estimated value in the second half of the culture according to the comparative example.

In the example shown in Fig. 10, a small deviation in the first half of the culture occurs with respect to the actual measured value.

That is, it is not possible to cope with a case in which the first half of culture is not corrected and the second half of culture is corrected in the single technique even though the relational expression of the technique of the related art described in Patent Literature 1 is corrected as shown in the comparative example.

On the other hand, in the present embodiment, a technique of separating a period from the start of culture to the end of culture for each phase and performing appropriate correction for each phase (performing estimation using a coefficient) is applied. Thus, according to the present embodiment, even though the culture conditions are different and the tendencies of deviation are different, it is possible to implement highly accurate viable cell count estimation during the entire period from seeding in cell culture (especially CHO cells) to death at a certain survival rate or less. Further, in the present embodiment, it is possible to obtain an effect that data other than the capacitance data used for the estimation is not required, while automatically selecting an optimal estimation technique for each phase. In the present embodiment, because the estimation technique in each of the classified phases is applied only to a phase in which the behavior of the capacitance can be considered to be steady, highly accurate estimation can be performed for general purposes.

Although an example in which the period is classified by performing principal component analysis or the like on the capacitance and the viable cell density in the impedance measured by the sensor 20 and a coefficient for estimating the number of viable cells present in a culture solution is set using the capacitance for each period is set has been described in the above-described example, the present invention is not limited thereto. The cell testing system 1 may be configured to classify periods by performing principal component analysis or the like on the capacitance, the conductance, and the viable cell density measured by the sensor 20 and use the impedance for each period to set a coefficient for estimating the number of viable cells present in the culture solution.

Although an example in which the control unit 24 and the calculation unit 26 are separated has been described in the above-described example, the control unit 24 and the calculation unit 26 may be integrally configured.

As described above, according to the present embodiment, it is possible to accurately estimate the number of viable cells in a prescribed period within the culture period from seeding in cell culture to death at a certain survival rate or less.

According to the present embodiment, the prescribed period is classified into a plurality of phases, a coefficient is set for each phase, and the number of viable cells is estimated using this coefficient. In this case, the number of viable cells can also be accurately estimated.

According to the present embodiment, for example, the estimation is performed using the coefficient of the first phase and the coefficient of the second phase in switching between the first phase and the second phase, so that the number of viable cells is also accurately estimated in case that the phase is switched.

In the present embodiment, because a previous coefficient is obtained using at least one of linear regression, partial least squares regression, and quadratic or higher regression, the amount of calculation is small.

In the present embodiment, because the phases are classified by performing the principal component analysis using the impedance and the actually measured number of viable cells, the phases can be classified with high accuracy.

In the present embodiment, because a phase during actual measurement is determined according to a probability distribution calculated for each item of data from the coefficient and the capacitance, the phase can be determined with high accuracy and the number of viable cells can be accurately estimated.

Also, all or a part of a process to be performed by the viable cell count estimation unit 28 may be performed by recording a program for implementing all or some of the functions of the viable cell count estimation unit 28 described in the present invention on a computer-readable recording medium and causing a computer system to read and execute the program recorded on the recording medium. The "computer system" used here may include an operating system (OS) and hardware such as peripheral devices. The "computer system" is also assumed to include a World Wide Web (WWW) system having a homepage providing environment (or displaying environment). The "computer-readable recording medium" refers to a storage device such as a flexible disc, a magneto-optical disc, a read-only memory (ROM), a portable medium such as a compact disc-digital versatile disc (CD-DVD), and a hard disk embedded in the computer system. Furthermore, the "computer-readable recording medium" is assumed to include a medium that holds a program for a certain period of time, such as a volatile memory (a random access memory (RAM)) inside a computer system serving as a server or a client in case that the program is transmitted via a network such as the Internet or a communication circuit such as a telephone circuit.

Also, the above-described program may be transmitted from a computer system storing the program in a storage device or the like to another computer system via a transmission medium or by transmission waves in a transmission medium. Here, the "transmission medium" for transmitting the program refers to a medium having a function of transmitting information, such as a network (communication network) like the Internet or a communication circuit (communication line) like a telephone circuit. Also, the above-described program may be a program for implementing some of the above-described functions. Further, the above-described program may be a program capable of implementing the above-described function in combination with a program already recorded on the computer system, i.e., a so-called differential file (differential program).

Although modes for carrying out the present invention have been described using embodiments, the present invention is not limited to the embodiments, and various modifications and substitutions can also be made without departing from the scope and spirit of the present invention.

## Claims

1. A cell testing device comprising:
an impedance sensor configured to measure impedance of a culture solution;
a storage unit configured to store a coefficient for estimating the number of viable cells present in the culture solution during a prescribed period using the impedance for each of a plurality of periods into which the prescribed period within a culture period from the start of cell culture to death is divided; and
a viable cell count estimation unit configured to acquire the impedance and estimate the number of viable cells using at least one of the acquired impedance and a coefficient for each period stored in the storage unit.

2. The cell testing device according to claim 1, wherein the storage unit stores the coefficient for each of a plurality of periods into which the prescribed period is divided based on the impedance measured by the impedance sensor, information about the number of viable cells, and an impedance measurement time.

3. The cell testing device according to claim 2,
wherein the viable cell count estimation unit performs principal component analysis with respect to the impedance, the information about the number of viable cells, and an elapsed time from a start time of the prescribed period in case that the impedance and the information about the number of viable cells are measured during a period from the start of the cell culture to the death,
wherein the viable cell count estimation unit divides the prescribed period into a plurality of periods based on a result of performing the principal component analysis,
wherein the viable cell count estimation unit obtains the coefficient for each of the periods into which the prescribed period is divided based on the result of performing the principal component analysis, and
wherein the viable cell count estimation unit causes the storage unit to store the coefficient obtained for each of the periods into which the prescribed period is divided.

4. The cell testing device according to claim 3, wherein the viable cell count estimation unit obtains the coefficient using at least one of linear regression, partial least squares regression, and quadratic or higher regression.

5. The cell testing device according to claim 3 or 4, wherein the viable cell count estimation unit increases the elapsed time for each prescribed period, obtains a ratio difference or a ratio between a change in the impedance and a change in the number of viable cells, obtains a branch point for an n^{th} (n is an integer of 1 or more) period and an (n+1)^{th} period in case that the difference or the ratio is outside of a prescribed range, and classifies the n^{th} period based on the obtained branch point.

6. The cell testing device according to any one of claims 1 to 5, wherein the viable cell count estimation unit obtains boundary information including a branch point for the plurality of periods and capacitance of the impedance at the branch point and causes the storage unit to store the obtained boundary information.

7. The cell testing device according to claim 6,
wherein the viable cell count estimation unit determines a period to which capacitance of the acquired impedance corresponds based on the boundary information stored in the storage unit, and
wherein the viable cell count estimation unit estimates the number of viable cells using the acquired impedance and the coefficient associated with the determined period.

8. A cell testing method for use in a cell testing device including an impedance sensor, a storage unit configured to store a coefficient for estimating the number of viable cells present in a culture solution during a prescribed period using impedance measured by the impedance sensor for each of a plurality of periods into which the prescribed period within a culture period from the start of cell culture to death is divided, and a viable cell count estimation unit, the cell testing method comprising:
measuring, by the impedance sensor, the impedance of the culture solution during the prescribed period; and
acquiring, by the viable cell count estimation unit, the impedance and estimating the number of viable cells using at least one of the acquired impedance and the coefficient for each period stored in the storage unit.

9. A program for causing a computer of a cell testing device including an impedance sensor, a storage unit configured to store a coefficient for estimating the number of viable cells present in a culture solution during a prescribed period using impedance measured by the impedance sensor for each of a plurality of periods into which the prescribed period within a culture period from the start of cell culture to death is divided, and a viable cell count estimation unit to:
acquire the impedance of the culture solution measured by the impedance sensor; and
estimate the number of viable cells using at least one of the acquired impedance and the coefficient for each period stored in the storage unit.

10. A computer-readable recording medium recording the program according to claim 9.
